# EUROPEAN PATENT SPECIFICATION

(11) **EP 3 286 270 B1**
(45) Date of publication and mention of the grant of the patent: **24.01.2024**
(21) Application number: 16783691.5
(22) Date of filing: 19.04.2016
(51) Int. Cl.: C09D 167/00, C09D 7/46, C09D 167/08, C07C 45/82, C09D 5/00

(54) **COATING COMPOSITION INCLUDING ALKYL OXIMES**
BESCHICHTUNGSZUSAMMENSETZUNG MIT ALKYLOXIMEN
COMPOSITION DE REVÊTEMENT COMPRENANT DES OXIMES D'ALKYLE

(30) Priority: 20.04.2015 US 201562149894 P
(43) Date of publication of application: 28.02.2018
(60) Divisional application: 23200389.7
(73) Proprietor: Advansix Resins & Chemicals LLC., Parsippany NJ 07054 (US)
(72) Inventor: ASIRVATHAM, Edward, Parsippany NJ 07054 (US)
(74) Representative: Crooks, Elizabeth Caroline
(86) International application number: PCT/US2016/028290
(87) International publication number: WO 2016/172108

(56) References cited:
- CA-A- 453 619
- CN-A- 103 193 672
- CN-A- 104 447 255
- US-A- 3 346 523
- US-A- 3 846 140
- US-A- 3 846 140
- US-A- 3 992 212
- US-A- 5 310 785
- US-A1- 2010 224 099
- PILEMAND CHARLOTTE ET AL: "Substitution of Cobalt Driers and Methyl Ethyl Ketoxime", ENVIRONMENTAL PROJECT NO.884, 1 January 2003 (2003-01-01), pages 1-119, XP055910374,

## Description

### FIELD

The present invention relates to coating compositions including an anti-skinning agent and, in particular, relates to high purity anti-skinning agents.

### BACKGROUND

Air-drying coating compositions, like paints, include unsaturated resins dissolved in an organic or aqueous solvent medium along with additives such as driers that impact the drying profile. The driers are catalysts used to accelerate the drying process, and may include multiple metal salts, such as metal octoates. For example, a paint composition may include cobalt or manganese salts to promote the autoxidation, zirconium salts for the polymerization or crosslinking of the resin, and calcium salts to control the film formation. These catalyst driers enable the paint to dry within a few hours. The cobalt and/or manganese salts are oxidation catalysts that play an important role in initiating the oxidation process.

Coating compositions such as alkyd paints that can dry in air are typically stored in cans. During storage, the paint may react with the air present over the composition to form a thin skin of cured paint on top of the paint. This unwanted reaction is referred to as the "skinning" of paints. This skinning phenomenon deteriorates the quality of the paint composition, impacts the strength of the driers, and negatively impacts the drying profile of the remaining paint. This skin formation is due to oxidative crosslinking of the resin and results in drying of the paint composition. Thus, anti-skinning agent additives are added to the coating composition to prevent the skinning of paints.

It is known that these anti-skinning agents not only behave as antioxidants to prevent oxidative crosslinking of the paint resin, but the anti-skinning agents also form complexes with the transition metal salt driers to avoid pre-mature drying inside the can. Without wishing to be held to any particular theory, it is believed that a complex formed between the anti-skinning agent and the transition metal salt driers is far less effective as a catalyst for the autoxidation polymerization process, and thus prevents premature drying of the paint in the can. When the coating composition is applied to a substrate, the surface area is increased, enabling the anti-skinning agent to evaporate. Evaporation of the anti-skinning agent destroys the complex between the anti-skinning agent and the metal salt driers, enabling the catalytic activity of the metal ions to be restored and the paint to dry.

Although several organic additives based on hydroxylamine derivatives, phenols, amino compounds and oximes of aldehydes and ketones have been used as anti-skinning agents, in practice, methyl ethyl ketoxime (MEKO) is typically regarded as the most effective and widely used anti-skinning agent. MEKO is known to form a complex with the primary metal salt driers to prevent premature drying in the can. MEKO will also evaporate easily to free the metal ion from the complex to facilitate the drying process once the paint is applied on a substrate. Additionally, MEKO provides benefits including low odor, low required dosage, applicability to a wide range of coatings, no yellowing or discoloration, no residue, no impact on the drying profile of the coating, and no impact on the performance of the coating, such as gloss, adhesion, or solvent resistance.

However, concerns have been raised relating to the toxicity of MEKO. MEKO has been identified as a skin sensitizer and a suspected carcinogen. In addition, the German Hazardous Substances Commission has reduced the Occupational Exposure Limit (OEL) for MEKO to a level of only 0.3 ppm. On February 2, 2016, the German Federal Institute for Occupational Safety and Health (BAuA) has notified the European Chemicals Agency (ECHA) of its intention to submit a proposal to revise the harmonized classification of MEKO from a Carcinogenic, Mutagenic, or Toxic for Reproduction (CMR) category Carc. 2 to the more severe CMR category Carc. 1B.

Other anti-skinning agents have been proposed as a replacement for MEKO, but each lacks one or more of the benefits of MEKO, as described above.

Improvements in the foregoing processes are desired.

US 3846140 discloses electrical resistor inks comprising a mixture of at least one pyropolymeric semi-conducting organic refractory oxide material, a vehicle consisting of a binder, and a suitable solvent, for use in forming resistors in electrical circuits used for thick film circuits.
Environmental Project No 884 2003 - Substitution of Cobalt Driers and Methyl Ethyl Ketoxime, November 2003 (Pilemand, Wallstrom, Hoffman, Poulsen) , Danish Environmental Protection Agency discloses replacements for MEKO in air-drying coatings.

### SUMMARY

The present disclosure provides a coating composition comprising:
at least one solvent;
at least one alkyd resin;
at least one drier; and
an anti-skinning composition capable of preventing oxidative crosslinking of the resin to form a skin, the anti-skinning composition comprising at least 92 wt.% of an alkyl oxime based on the total weight of the anti-skinning composition, wherein the alkyl oxime is selected from 2-pentanone oxime and 3-methyl-2-butanone oxime; and wherein the at least one drier is an oxidation catalyst selected from cobalt esters and manganese esters.

In one embodiment, the anti-skinning composition is a high-purity 2-pentanone oxime. In one more particular embodiment, the anti-skinning composition has a purity of at least 92 wt.% 2-pentanone oxime. In one more particular embodiment, the anti-skinning composition has a purity of at least 98 wt.% 2-pentanone oxime. In another more particular embodiment, the anti-skinning composition comprises less than 0.5 wt.% methyl isobutyl ketoxime, based on the total weight of the anti-skinning composition. In another more particular embodiment, the coating composition comprises less than 0.06 wt.% methyl isobutyl ketoxime, based on the total weight of the composition. Preferably, the anti-skinning composition has a purity of at least 98 wt.% 2-pentanone oxime and has less than 0.5 wt.% methyl isobutyl ketoxime based on the total weight of the anti-skinning composition, preferably less than 0.06 wt.% methyl isobutyl ketoxime, based on the total weight of the composition. In one more particular embodiment, the anti-skinning agent consists essentially of 2-pentanone oxime. In another more particular embodiment, the anti-skinning agent is a high-purity 3-methyl-2-butanone oxime.

In one embodiment, a coating composition is provided. The coating composition comprises at least one solvent, at least one alkyd resin, at least one drier, and an anti-skinning composition capable of preventing oxidative crosslinking of the resin to form a skin, wherein the anti-skinning composition comprises at least 92 wt.%, or more particularly at least 98 wt.%, of an alkyl oxime selected from 2-pentanone oxime and 3-methyl-2-butanone oxime.

In one more particular embodiment, the anti-skinning composition comprises a high-purity 2-pentanone oxime and methyl ethyl ketoxime. In one more particular embodiment, the anti-skinning composition has less than 0.5 wt.% methyl isobutyl ketoxime, based on the total weight of the anti-skinning composition. In another more particular embodiment, the coating composition has less than 0.06 wt.% methyl isobutyl ketoxime, based on the total weight of the composition. In one more particular embodiment, the anti-skinning composition consists essentially of 2-pentanone oxime and methyl ethyl ketoxime.

In one embodiment, a coating composition is provided including at least one solvent, at least one alkyd resin, at least one drier, and an anti-skinning composition capable of preventing oxidative crosslinking of the resin to form a skin, wherein the anti-skinning composition comprises at least 92 wt.%, or more particularly at least 98 wt.%, based on the total weight of the anti-skinning composition, of an alkyl oxime selected from 2-pentanone oxime and 3-methyl-2-butanone oxime.

In one more particular embodiment, the anti-skinning composition comprises at least 92 wt.% 2-pentanone oxime. In one more particular embodiment, the anti-skinning composition comprises at least 98 wt.% 2-pentanone oxime. In a more particular embodiment, the anti-skinning composition comprises at least 99 wt.% 2-pentanone oxime. In another more particular embodiment, the anti-skinning composition comprises at least 99.5 wt.% 2-pentanone oxime. In still another more particular embodiment, the anti-skinning composition comprises at least 99.9 wt.% 2-pentanone oxime. In another more particular embodiment, the anti-skinning composition is a high-purity 3-methyl-2-butanone oxime.

In one embodiment, an anti-skinning composition capable of preventing oxidative crosslinking of the resin to form a skin is provided wherein the anti-skinning composition comprises at least 92 wt.%, or more particularly at least 98 wt.%, of an alkyl oxime selected from 2-pentanone oxime and 3-methyl-2-butanone oxime. In one more particular embodiment, the anti-skinning composition comprises 2-pentanone oxime provided in combination with a solvent selected from xylene, mineral spirits, alcohol, and water. In a more particular embodiment, the anti-skinning composition comprises at least 92 wt.% 2-pentanone oxime. In another more particular embodiment, the anti-skinning composition comprises at least 98 wt.% 2-pentanone oxime. In another embodiment, a coating composition comprising either of the above anti-skinning compositions is provided. In another more particular embodiment, the anti-skinning composition is a high-purity 3-methyl-2-butanone oxime.

In a more particular embodiment of any of the above embodiments, the anti-skinning composition comprises less than 0.5 wt.% methyl isobutyl ketoxime, based on the total weight of the anti-skinning composition. In another more particular embodiment of any of the above embodiments, the anti-skinning composition comprises less than 0.3 wt.% methyl isobutyl ketoxime, based on the total weight of the anti-skinning composition. In another more particular embodiment of any of the above embodiments, the anti-skinning composition comprises less than 0.1 wt.% methyl isobutyl ketoxime, based on the total weight of the anti-skinning composition.

In a more particular embodiment of any of the above embodiments, the anti-skinning agent comprises at least 92 wt.% 2-pentanone oxime, preferably at least 98 wt.% 2-pentanone oxime, at least 99 wt.% 2-pentanone oxime, at least 99.5 wt.% 2-pentanone oxime, and or at least 99.9 wt.% 2-pentanone oxime, based on the total weight of the composition. In another more particular embodiment of any of the above embodiments, the anti-skinning composition comprises less than 0.5 wt.% methyl isobutyl ketoxime, preferably less than 0.3 wt.% methyl isobutyl ketoxime, or less than 0.1 wt.% methyl isobutyl ketoxime, based on the total weight of the composition. In a more particular embodiment of any of the above embodiments, the anti-skinning composition comprises at least 92 wt.% 2-pentanone oxime, preferably at least 98 wt.% 2-pentanone oxime, at least 99 wt.% 2-pentanone oxime, at least 99.5 wt.% 2-pentanone oxime, or at least 99.9 wt.% 2-pentanone oxime and the anti-skinning composition comprises less than 0.5 wt.% methyl isobutyl ketoxime, less than 0.3 wt.% methyl isobutyl ketoxime, or less than 0.1 wt.% methyl isobutyl ketoxime, based on the total weight of the anti-skinning composition.

In the coating compositions of the present invention at least one drier is present that comprises cobalt esters and manganese esters. In another more particular embodiment of any of the above embodiments, the at least one solvent comprises at least one solvent selected from the group consisting of: xylene, mineral spirits, alcohol, and water, and combinations thereof. In another more particular embodiment of any of the above embodiments, the coating composition further includes at least one additive selected from the group consisting of: fillers, pigments, surfactants, stabilizers, thickeners, emulsifiers, texture additives, adhesion promoters, biocides, and additives to modify viscosity or finished appearance.

In a more particular embodiment of any of the above embodiments, the coating composition has a drying time at least as short as a similar composition having the same components except that the weight percentage of 2-pentanone oxime and/or 3-methyl-2-butanone oxime is replaced with an equivalent weight percentage of methyl ethyl ketoxime.

The above mentioned and other features of the invention, and the manner of attaining them, will become more apparent and the invention itself will be better understood by reference to the following description of embodiments of the invention taken in conjunction with the accompanying drawings.

### BRIEF DESCRIPTION OF THE DRAWINGS

FIG. 1 shows the oximation of 2-pentanone to form 2-pentanone oxime ("2-PO") using hydroxylamine.
FIG. 2 shows the production of 2-pentanone from acetaldehyde and acetone.
FIG. 3 shows the formation of methyl isobutyl ketone ("MIBK") from the self-condensation of acetone.
FIG. 4 shows the oximation of MIBK to methyl isobutyl ketoxime ("MIBKO") using hydroxylamine.
FIG. 5 shows an exemplary process for producing a 2-PO product.
FIG. 6 shows an exemplary process for producing a coating composition including a high-purity 2-PO product.

### DETAILED DESCRIPTION

Alkyl oximes having five carbon atoms include 2-pentanone oxime and 3-methyl-2-butanone oxime. As shown below, it has been discovered that high-purity 2-pentanone oxime and 3-methyl-2-butanone oxime function as effective anti-skinning agents.

2-pentanone oxime ("2-PO"), also known as methyl propylketoxime, is an alkyl oxime having the following formula (I):

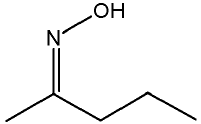

As shown below, it has been discovered that high-purity 2-PO functions as an effective anti-skinning agent. 2-PO has a vapor pressure similar to that of MEKO. In addition, 2-PO provides similar benefits to MEKO, including low required dosage, applicability to a wide range of coatings, no yellowing or discoloration, no residue, and no impact on the performance of the coating, such as gloss, adhesion, or solvent resistance. Additionally, high-purity 2-PO, which includes relatively low levels of methyl isobutyl ketoxime (MIBKO) as described below, provides similar drying profiles to MEKO, as well as low odor.

However, 2-PO has a positive toxicology profile compared to MEKO.

The saturated vapor concentration of MEKO is 1350 ppm, while that of 2-PO is only 300 ppm, or less than 25% of that of MEKO. The lower saturated vapor concentration provides a lower inhalation risk for 2-PO compared to MEKO.

For dermal irritation, MEKO is a slight irritant, while 2-PO produces no irritation. For eye irriation, MEKO is categorized as causing serious eye damage (code H318), while 2-PO is categorized as only causing serious eye irritation (code H319).

MEKO is classified as a sensitizer (R43), while 2-PO is not a sensistizer.

MEKO has an effective concentration of 50% growth inhabitation (EC₅₀) for algae of only 7 ppm, while the corresponding EC₅₀ of 2-PO for algae is 88 ppm. MEKO has an lethal concentration of 50% mortality (LC₅₀) for fish of only 48 ppm, while the corresponding LC₅₀ of 2-PO for fish is greater than 100 ppm.

The positive toxicology profile of 2-PO compared to MEKO was unexpected.

3-methyl-2-butanone oxime, also known as methyl isopropyl ketoxime, is a alkyl oxime having the following formula (II):

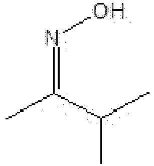

3-methyl-2-butanone oxime has a vapor pressure at 20°C of less than 130 Pa (0.975 mm Hg), compared to about 213 Pa (about 1.60 mm Hg) for 2-PO and 267 Pa (2.00 mm Hg) for MEKO.

### 1. Typical production of 2-pentanone oxime

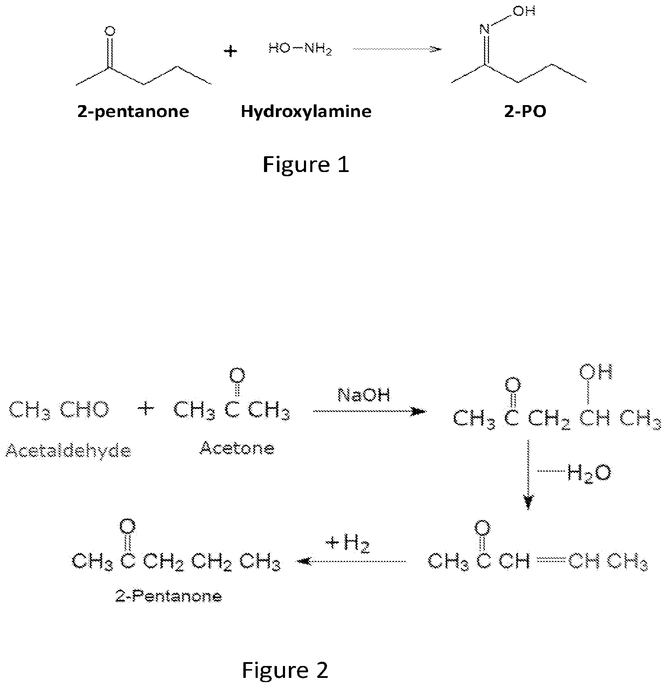

2-pentanone oxime is produced from the oximation of 2-pentanone with hydroxylamine, as shown in Figure 1. 2- pentanone is commercially produced from acetaldehyde and acetone via aldol condensation, dehydration and hydrogenation, as shown by the reaction summarized in Figure 2.

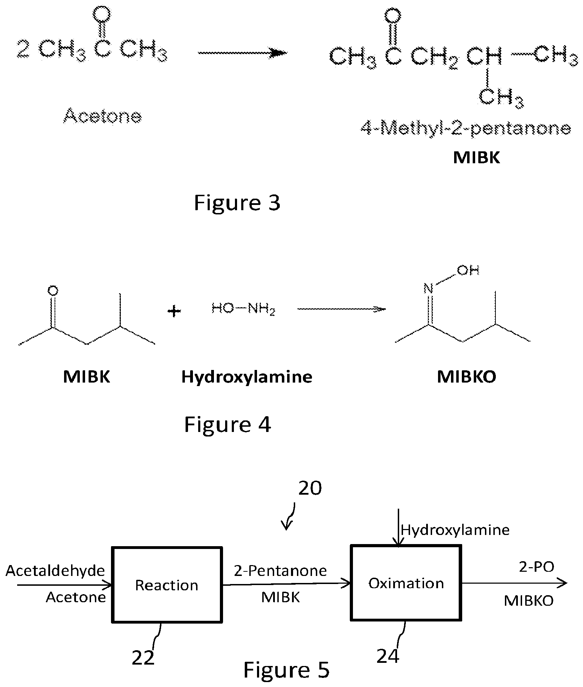

However, in the reaction shown in Figure 2, it is known and unavoidable that a portion of the acetone reactant will undergo self-condensation following the same reaction pathway to form methyl isobutyl ketone (MIBK), also known as 4-methyl-2- pentanone. This side reaction is shown in Figure 3. As a result, the 2-pentanone product produced by the reaction shown in Figure 1 will contain at least some MIBK, which may be as much as 8-10 wt.% of the total mixture of 2-pentanone and MIBK.

However, in direct oximation of a 2-pentanone feed that also includes MIBK, the hydroxylamine also reacts with the MIBK product in an oximation reaction as shown in Figure 4 to form methyl isobutyl ketoxime (MIBKO).

The above reactions are summarized by process 20 shown in Figure 5. As shown in Figure 5, acetaldehyde and acetone are reacted in reaction 22 to form 2-pentanone by the reaction mechanism shown in Figure 2. However, a portion of the acetone undergoes self-condensation to form MIBK, as shown in Figure 3. Accordingly, the product of reaction 22 includes a mixture of 2-pentanone and MIBK, as shown in Figure 5. The oximation 24 of this mixture with hydroxylamine results in oximation of 2-pentanone to form 2-PO, as shown in Figure 1, and the oximation of MIBK to form MIBKO, as shown in Figure 4. The product of oximation 24 is a mixture of 2-PO and MIBKO. In this manner, direct oximation of 2-pentanone to form a 2-PO product will include a significant amount of MIBKO, due to the unavoidable presence of at least some MIBK in the 2-pentanone reactant.

The presence of methyl isobutyl ketoxime (MIBKO) in coating compositions is undesirable for several reasons.

First, the vapor pressure of MIBKO, about 0.13 hPa, is significantly lower than that of 2-pentanone oxime, about 2.14 hPa, at 20°C. Like MEKO and 2-PO, MIBKO will also form complexes with the transition metal salt driers. However, due to the significantly lower vapor pressure of MIBKO, complexes formed between MIBKO and the transition metal salt driers will be much more stable and retard the drying process to a significantly greater extent than complexes formed between 2-PO and the transition metal salt driers, leading to an undesirably lengthy drying time for the coating composition.

Second, MIBKO is known to have very strong objectionable odor, which is undesirable in coating formulations such as alkyd paints. The objectionable odor would negatively affect the desirability of use of alkyd paints and other coating compositions which include MIBKO, especially for indoor applications and for do-it-yourself (DIY) customers.

Removal of MIBKO from 2-PO by distillation is relatively difficult. Both MIBKO and 2-PO are temperature sensitive materials, which are subject to thermal decomposition below their respective atmospheric boiling points. As a result, vacuum distillation is required for the distillation of these oximes. In an apparatus to produce MIBKO substantially free of 2-PO in the bottoms, an operating pressure less than 6.67 kPa (50 mm Hg) would be required. Additionally, the MIBKO rich bottoms product has limited economic value, and incineration of the bottoms product would be required for disposal. The methods disclosed herein avoid the need to remove MIBKO from 2-PO by separating MIBK from 2-pentanone prior to the oximation reaction.

### 2. Coating compositions including a high-purity 2-PO or high-purity 3-methyl-2-butanone oxime

In one embodiment, a coating composition is provided. The coating composition is an alkyd paint composition. The coating composition includes an anti-skinning agent in the form of a high-purity alkyl oxime, or more particularly an alkyl oxime having 5 carbon atoms, such as 2-PO or high-purity 3-methyl-2-butanone oxime.

The term "high purity 2-PO" is generally used herein to refer to an anti-skinning composition which comprises at least 92 wt.%, preferably at least 98 wt.%, at least 99 wt.%, at least 99.5 wt.%, or at least 99.9 wt.% 2-PO by weight of the anti-skinning agent composition. Preferably the "high purity 2-PO" comprises less than 0.5 wt.% methyl isobutyl ketoxime, less than 0.3 wt.% methyl isobutyl ketoxime, or less than 0.1 wt.% methyl isobutyl ketoxime of the total anti-skinning agent composition.

The term "high purity 3-methyl-2-butanone oxime" is generally used herein to refer to an anti-skinning composition which at least 92 wt.%, least 98 wt.%, at least 99 wt.%, 99.5 wt.%, or at least 99.9 wt.% 3-methyl-2-butanone oxime by weight of the anti-skinning agent composition.

In some embodiments, the coating composition includes one or more components selected from the group consisting of: one or more binders, one or more fillers, one or more pigments, one or more solvents, and one or more driers. For example, the coating composition may include one or more solvents and one or more driers, or may include one or more binders and one or more pigments, or may include one or more solvents, one or more driers, and one or more pigments. Exemplary solvents include xylene, mineral spirits, alcohol, and water.

In some embodiments, the coating composition has a similar drying time or a similar drying rate than that of a similar coating composition in which the 2-pentanone oxime, 3-methyl-2-butanone oxime, or mixture thereof is replaced on an equal weight basis with MEKO. In some embodiments, the coating composition has a faster drying time and/or a greater drying rate than that of a similar coating composition in which the 2-pentanone oxime, 3-methyl-2-butanone oxime, or mixture thereof is replaced on an equal weight basis with MEKO.

### a. Anti-skinning agent

In one embodiment, the coating composition includes at least one high-purity alkyl oxime having 5 carbon atoms as an anti-skinning agent, such as 2-PO or high-purity 3-methyl-2-butanone oxime. As disclosed herein, the purity levels of the anti-skinning agent are expressed as a weight percentage, either as a weight percentage of the anti-skinning chemical compound in connection with a particular anti-skinning agent, or in connection with an anti-skinning agent composition including one or more particular anti-skinning agent chemical compounds. Exemplary anti-skinning chemical compounds include 2-PO, 3-methyl-2-butanone oxime, and MEKO. Exemplary impurities include MIBKO.

In one embodiment, the high-purity alkyl oxime is 2-PO. In a more particular embodiment, the purity level of 2-PO is at least 92 wt.%, greater than 97 wt,%, at least 98 wt.%, greater than 98 wt.%, at least 99 wt.%, greater than 99 wt.%, at least 99.5 wt.%, greater than 99.5 wt.%, or at least 99.9 wt.%, or within any range defined between any two of the foregoing values, such as at least 92 wt.% to 99.9 wt.%, greater than 97 wt.% to 99.9 wt.%, greater than 98 wt.% to 99.9 wt.%, 99 wt.% to 99.9 wt.%, greater than 99 wt.% to 99.9 wt.%, 99.5 wt.% to 99.9 wt.% or greater than 99.5 wt.% to 99.9 wt.%.

In one embodiment, the high purity 2-PO comprises no more than 2 wt.% MIBKO, no more than 1.5 wt.% MIBKO, no more than 1 wt.% MIBKO, no more than 0.5 wt.% MIBKO, no more than 0.3 wt.% MIBKO, or no more than 0.1 wt.% MIBKO.

In some embodiments, the composition includes as little as 0.1 wt.%, 0.2 wt.%, 0.25 wt.%, 0.3 wt.%, 0.35 wt.%, 0.4 wt.%, as great as 0.5 wt.%, 0.99 wt.% 1.0 wt.%, 1.25 wt.%, 1.5 wt.%, 2 wt.%, 3 wt.%, of the high-purity 2-PO based on the total weight of the composition, or within any range defined between any two of the foregoing values such as 0.1 wt.% to 3 wt.%, 0.2 wt.% to 2 wt.%, 0.25 wt.% to 1.5 wt.%, 0.3 wt.% to 1.25 wt.%, 0.35 wt.% to 0.99 wt.%, or 0.4 wt.% to 0.5 wt.%. It will also be appreciated that the composition may comprise 0.2 wt.% to 0.5 wt.%, 0.2 wt.% to 0.4 wt.%, 0.25 wt. % to 1.0 wt.% or 0.5 wt.% to 0.99 wt.% of the total anti-skinning agent based on the total weight of the composition.

In some embodiments, the composition includes the same or less 2-PO as an anti-skinning agent than the amount of MEKO in a similar composition to achieve at least one of the same drying time and the same drying rate.

In some embodiments, the composition comprises no more than 0.06 wt.% MIBKO, preferably no more than 0.05 wt.% MIBKO, no more than 0.02 wt.% MIBKO, no more than 0.01 wt.% MIBKO, no more than 0.005 wt.% MIBKO, no more than 0.002 wt.% MIBKO, no more than 0.001 wt.% MIBKO, no more than 0.0005 wt.% MIBKO, no more than 0.0002 wt.% MIBKO, or no more than 0.0001 wt.% MIBKO, based on the total weight of the composition.

In another embodiment, the anti-skinning agent includes a mixture of 2-PO and MEKO. In one embodiment, the anti-skinning agent consists essentially of 2-pentanone oxime and methyl ethyl ketoxime In some embodiments, the composition includes as little as 0.1 wt.%, 0.2 wt.%, 0.3 wt.%, 0.35 wt.%, 0.4 wt. %, 0.5 wt.%, as great as 1.0 wt.%, 1.25 wt.%, 1.5 wt.%, 2 wt.%, 3 wt.%, of the total anti-skinning agent based on the total weight of the composition, or within any range defined between any two of the foregoing values such as 0.1 wt.% to 3 wt.%, 0.2 wt.% to 2 wt.%, 0.25 wt.% to 1.5 wt. %, 0.3 wt.% to 1.35 wt.%, 0.3 wt.% to 0.99 wt.%, or 0.4 wt.% to 0.5 wt.%. It will also be appreciated that the composition may comprise 0.25 wt. % to 1.0 wt.% or 0.2 wt.% to 1.5 wt.% of the total anti-skinning agent based on the total weight of the composition.

In one embodiment, the high-purity alkyl oxime is 3-methyl-2-butanone oxime. In a more particular embodiment, the purity level of 3-methyl-2-butanone oxime is at least 92 wt.%, greater than 97 wt,%, at least 98 wt.%, greater than 98 wt.%, at least 99 wt.%, greater than 99 wt.%, at least 99.5 wt.%, greater than 99.5 wt.%, or at least 99.9 wt.%, or within any range defined between any two of the foregoing values, such as at least 92 wt.% to 99.9 wt.%, greater than 97 wt.% to 99.9 wt.%, greater than 98 wt.% to 99.9 wt.%, 99 wt.% to 99.9 wt.%, greater than 99 wt.% to 99.9 wt.%, 99.5 wt.% to 99.9 wt.% or greater than 99.5 wt.% to 99.9 wt.%.

In some embodiments, the composition includes as little as 0.1 wt.%, 0.2 wt.%, 0.25 wt.%, 0.3 wt.%, 0.35 wt.%, 0.4 wt.%, as great as 0.5 wt.%, 0.99 wt.% 1.0 wt.%, 1.25 wt.%, 1.5 wt.%, 2 wt.%, 3 wt.%, of the high-purity 3-methyl-2-butanone oxime based on the total weight of the composition, or within any range defined between any two of the foregoing values such as 0.1 wt.% to 3 wt.%, 0.2 wt.% to 2 wt.%, 0.25 wt.% to 1.5 wt.%, 0.3 wt.% to 1.25 wt.%, 0.35 wt.% to 0.99 wt.%, or 0.4 wt.% to 0.5 wt.%. It will also be appreciated that the composition may comprise 0.2 wt.% to 0.5 wt.%, 0.2 wt.% to 0.4 wt.%, 0.25 wt. % to 1.0 wt.% or 0.5 wt.% to 0.99 wt.% of the total anti-skinning agent based on the total weight of the composition.

In some embodiments, the composition includes the same or less 3-methyl-2-butanone oxime as an anti-skinning agent than the amount of MEKO in a similar composition to achieve at least one of the same drying time and the same drying rate.

In another embodiment, the anti-skinning agent includes a mixture of 3-methyl-2-butanone oxime and MEKO. In one embodiment, the anti-skinning agent consists essentially of 3-methyl-2-butanone oxime. In some embodiments, the composition includes as little as 0.1 wt.%, 0.2 wt.%, 0.3 wt.%, 0.35 wt.%, 0.4 wt. %, 0.5 wt.%, as great as 1.0 wt.%, 1.25 wt.%, 1.5 wt.%, 2 wt.%, 3 wt.%, of the total anti-skinning agent based on the total weight of the composition, or within any range defined between any two of the foregoing values such as 0.1 wt.% to 3 wt.%, 0.2 wt.% to 2 wt.%, 0.25 wt.% to 1.5 wt. %, 0.3 wt.% to 1.35 wt.%, 0.3 wt.% to 0.99 wt.%, or 0.4 wt.% to 0.5 wt.%. It will also be appreciated that the composition may comprise 0.25 wt. % to 1.0 wt.% or 0.2 wt.% to 1.5 wt.% of the total anti-skinning agent based on the total weight of the composition.

In another embodiment, the anti-skinning agent includes a mixture of 2-PO and 3-methyl-2-butanone oxime.

In another embodiment, the anti-skinning agent includes a mixture of 2-PO, 3-methyl-2-butanone oxime, and MEKO.

### b. Binders

In one embodiment, the coating composition includes one or more binders. Exemplary binders include various types of alkyd resins. Exemplary alkyd resins include alkyd resins having short, medium, long, and very long oil length. The term "alkyd resin" also includes alkyds modified with other resins such as acrylic, epoxy, phenolic, urethane, polystyrene, silicone, rosin and rosin ester alkyds, and bio-alkyds, such as Setal 900 SM-90, in which the polyester segment is derived from renewable acids and esters.

In some embodiments, the composition includes as little as 1 wt.%, 5 wt.%, 10 wt.%, 15 wt.%, 25 wt.%, 30 wt.%, as great as 35 wt.%, 40 wt.%, 50 wt.%, 60 wt.%, of the one or more binders based on the total weight of the composition, or within any range defined between any two of the foregoing values such as 1 wt.% to 60 wt.%, 5 wt.% to 50 wt.%, 10 wt.% to 40 wt.%, 15 wt.% to 35 wt.% or 25 wt.% to 30 wt.%. It will also be appreciated that the composition may comprise 5 wt.% to 60 wt.%, 5 wt.% to 10 wt.%, 20 wt.% to 30 wt.%, or 35 wt.% to 60 wt.% of the one or more binders based on the total weight of the composition.

### c. Solvents

In one embodiment, the coating composition includes one or more aqueous or organic solvents like mineral spirits and alcohols. Exemplary solvents include hydrocarbon solvent or their blends. The hydrocarbon solvents may be aliphatic or aromatic solvents. Examples of organic solvents are petroleum distillates such as pentane, hexane, petroleum naptha, heptanes, and 90 solvent (an aliphatic solvent with a flash point of 60 °C (140°F)). Aromatic solvents include xylene, toluene, Aromatic 100 and other suitable aromatic solvents. The term "mineral spirits", also known as "white spirits", encompasses compositions which comprise a mixture of C₇ to C₁₂ aliphatic and alicyclic hydrocarbons, and in a more particular embodiment comprises 15 wt.% to 20 wt.% or less of C₇ to C₁₂ aromatic hydrocarbons, based on the total weight of the composition. Mineral spirits include mixtures or blends of paraffins, cycloparaffins, and aromatic hydrocarbons. Typical mineral spirits have boiling ranges between about 150°C and 220°C, are generally clear water-white liquids, are chemically stable and non-corrosive, and possess a mild odor. Exemplary mineral spirits include Low Aromatic White Spirit (LAWS) such as Shell Sol 15 (CAS 64742-88-7) and ShellSol H (CAS 64742-82-1). The term "alcohol" encompasses is intended to encompass C₁ to C₁₂ alcohols, including C₁ to C₁₂ straight chain and branched alcohols. Exemplary alcohols include triethylene glycol (CAS 112-27-6) and diethylene glycol ethylether (CAS 111-90-0). In a more particular embodiment, the coating composition comprises a solvent selected from the group consisting of xylene, mineral spirits, alcohol, water, and combinations thereof.

In some embodiments, the composition includes as little as 5 wt.%, 10 wt.%, 15 wt.%, 17 wt.%, 20 wt.%, 25 wt.%, as great as 30 wt.%, 40 wt.%, 60 wt.%, of the one or more solvents based on the total weight of the composition, or within any range defined between any two of the foregoing values such as 5 wt.% to 60 wt.%, 10 wt.% to 40 wt.%, or 25 wt.% to 30 wt.%. It will also be appreciated that the composition may comprise 10 wt.% to 20 wt.%, 25 wt.% to 35 wt.%, or 40 wt.% to 60 wt.% of the one or more solvents based on the total weight of the composition.

### d. Driers

The coating composition includes one or more driers. The driers are catalysts used to accelerate the drying process. The at least one drier is an oxidation catalyst selected from cobalt esters or manganese esters that control the film formation. Driers enable the paint to fully dry within a few hours, such as within three hours, two hours, or less, after application to a surface. Cobalt or manganese esters are oxidation catalysts that play a role in initiating the oxidation process, and include esters of C₆-C₁₉ branched fatty acids. Examples are Cobalt 2-ethylhexanoate, propionate, Neodecanoate, Naphthenate, Cobalt embeded polymer product called ECOS ND15 available from Umicore, Manganese Octoate, Manganese-amine complex called Nuodex Drycoat available from Huntsman.

In some embodiments, the drier composition includes as little as 0.1 wt.%, 0.3 wt.%, 0.6 wt.%, as great as 1.0 wt.%, 1.2 wt.%, 1.5 wt.%, 3.5 wt.%, 6.0 wt.%, of the one or more driers based on the total weight of the composition, or within any range defined between any two of the foregoing values such as 0.1 wt.% to 6 wt.%, 0.3 wt.% to 3.5 wt.% or 0.6 wt.% to 1.5 wt.%. It will also be appreciated that the composition may comprise 0.1 wt.% to 1.0 wt.%, 1.0 wt.% to 3.0 wt.%, or 3.0 wt.% to 6 wt.% of the one or more driers based on the total weight of the composition.

### e. Additives

In one embodiment, the coating composition includes one or more additives such as fillers, pigments, surfactants, stabilizers, thickeners, emulsifiers, texture additives, adhesion promoters, biocides, flow promoters, dispersing agents, and additives to modify viscosity or finished appearance.

In some embodiments, the composition includes as little as 0.1 wt.%, 0.5 wt.%, 1.0 wt.%, 1.5 wt.%, as great as 2.0 wt.%, 5.0 wt.%, 10.0 wt.%, 20 wt.%, 25 wt.%, 30 wt.%, of the one or more additives based on the total weight of the composition, or within any range defined between any two of the foregoing values. such as 0.1 wt.% to 10 wt.%, 1.0 wt.% to 5.0 wt.% or 1.5 wt.% to 2.0 wt.%. It will also be appreciated that the composition may comprise 0.1 wt.% to 1.5 wt.%, 1.5 wt.% to 5.0 wt.%, or 5.0 wt.% to 10.0 wt.% of the one or more additives based on the total weight of the composition.

In one embodiment, the coating composition includes one or more fillers to thicken and increase the volume of the composition. Exemplary fillers include titanium oxide, calcium carbonate, clays, and talc.

In one embodiment, the coating composition includes one or more pigments to color the composition and/or provide opacity to the composition. As used herein, pigment includes both inorganic metal oxides and organic Color pigments. Exemplary pigments include metal oxides such as titanium oxide and iron oxides, Zinc Chromates, Chromium oxides, Cadmium sulfides, Azurite (made from kaolin, Sodium carbonate, sulfur and carbon), Lithopone (zinc sulfide and Barium sulfate blend). Examples of organic color pigments are Phthalocyanine Blue (alpha & beta), Dinitraniline Orange (PO-5), Perylene Red, Toluidine Red (PR-3), Diarylide Yellow (PY-12,13) and Quinacridone Red (PV-19)

In some embodiments, the composition includes as little as 0 wt.%, 1 wt.%, 5 wt.%, 10 wt.%, as great as 15 wt.%, 20 wt.%, 25 wt.%, 30 wt.% of a filler and/or pigment, such as titanium dioxide based on the total weight of the composition, or within any range defined between any two of the foregoing values such as 0 wt.% to 30 wt.%, 5 wt.% to 25 wt.%, or 15 wt.% to 30 wt.%.

In one embodiment, the coating composition includes one or more additives selected from the group consisting of surfactants, stabilizers, thickeners, emulsifiers, texture additives, adhesion promoters, biocides, and additives to modify viscosity or finished appearance.

### 3. Process for producing a coating composition

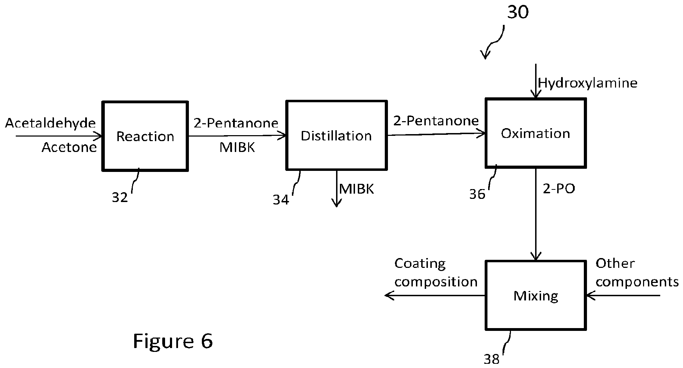

Referring next to Figure 6, a process 30 for producing a coating composition is provided. The process includes distilling 34 the MIBKO from the 2-pentanone after the reaction process 32 and prior to the oximation reaction 36. By performing this step prior to addition of the hydroxylamine, the MIBK is removed from the 2-pentanone reactant stream, and does not undergo the oximation reaction shown in Figure 5. This in turn prevents the formation of the undesirable MIBKO product component as a part of the 2-PO product during the oximation reaction 36. A coating composition is then formed by combining 38 the resulting high-purity 2-PO product with other components, such as resins, fillers, pigments, solvents, driers, and other additives as described above.

### 4. Examples

The coating formulations below were evaluated according to the following test methods.

Film formation of the coating was determined by visual observation. If a film was observed, it was removed and the film thickness was measured. Film formation may be observed after two months at room temperature or after accelerated aging for four weeks at 50°C.

Drying times were determined using a drying time recorder according to ASTM D5895, standard test methods for evaluating drying or curing during film formation of organic coatings using mechanical recorders.

An initial stage dry time was determined 1 day following paint preparation. A coating sample was applied to a Leneta sheet at a fixed humid thickness. The drying time recorder was immediately placed on the wet film and the stylus lowered on to the wet paint. After the stylus has moved across the sheet at a constant speed, the stages of drying time are determined by examining the sheet. Stage I is a set-to-touch time; Stage II is a tack-free time, Stage III is a dry-hard time, and Stage IV is a dry-through time.

A post-aging drying time was determined following storage of a sample at an elevated temperature of 50°C for four weeks to model accelerated aging of the sample. The samples were placed in closed containers having a large air volume compared to the coating formulation volume to further accelerate the aging process. After four weeks, the samples were visually inspected for film formation. A post-aging drying time was determined using the same method as for the initial stage dry time.

### a) Example 1: Glossy white one-coat finish using various purity anti-skinning agents

Glossy white one-coat finish = EU SF 3.11 //cobalt drier formulations were prepared according to the weight percentages shown in Table 1:

**Table 1: Example 1 formulations (wt.%)**

| **Part** | **Component** | **Function** | **Wt. %** |
|---|---|---|---|
| A | SETAL AF 681 TB | Alkyd resin | 30 |
| A | White spirit D60 | Solvent | 10 |
| A | Borchi Gen 911 | Wetting and dispersing agent | 2.6 |
| A | Kronos 2310 | Titanium dioxide | 26 |
| A | Borchi Gol E, 50% in Solvesso 100 | Flow promoter and release agent | 1 |
| A | Octa-Soligen Calcium 10, basic | Calcium-containing drier | 0.5 |
| B | SETAL AF 681 TB | Alkyd resin | 20 |
| B | White spirit D60 | Solvent | 7.6 |
| B | Borchi Gol OL 17, 10% in xylene | Flow promoter and release agent | 1 |
| B | Octa-Soligen Zirconium 12 | Zirconium-containing drier | 0.5 |
| B | Octa-Soligen 69 | Cobalt-containing drier | 0.3 |
| C | Anti-skinning agent | Anti-skinning agent | 0.5 |

The Part A components were subjected to ball milling at 3500 rpm for 45 minutes prior to cooling. The Part B components were then incorporated under gentle agitation/homogenization for 5 minutes.

The Part C anti-skin additive for each formulation was varied according to the formulations provided in Table 2. The Part C anti-skin additive was incorporated into Part A and Part B one day following preparation of Part A and Part B for Ex. 1-3 and Ref. Ex. 9, two days for Ex. 4-7, and seven days following preparation for Ex. 8.

**Table 2: Anti-skinning agent composition (wt.%)**

| **Formulation** | **% MEKO** | **% 2-PO** | **% MIBKO** |
|---|---|---|---|
| Ex. 1 | - | 92% | 8% |
| Ex. 2 | - | 94% | 6% |
| Ex. 3 | - | 96% | 4% |
| Ex. 4 | - | 97% | 3% |
| Ex. 5 | - | 98% | 2% |
| Ex. 6 | - | 99% | 1% |
| Ex. 7 | - | 99.5% | 0.50% |
| Ex. 8 | - | 99.7% | 0.30% |
| Ref. Ex. 9 | 100% | - | - |

In addition, a Ref. Ex. 10 formulation was similarly prepared, but lacking any anti-skinning agent.

Each formulation was subjected to an additional initial drying test, in which the times for Stage I "Set-to-Touch," Stage II "Tack-free," and Stage IV "Dry-through" were determined. The results are provided in Table 4

**Table 3: Initial drying times for Example 1**

| **Formulation** | **Stage I Dry Time (hr)** | **Stage II Dry Time (hr)** | **Stage IV Dry Time (hr)** |
|---|---|---|---|
| Ex. 1 | 1.61 | 3.77 | 5.44 |
| Ex. 2 | 1.57 | 3.69 | 5.25 |
| Ex. 3 | 1.72 | 3.92 | 5.59 |
| Ex. 4 | 1.56 | 3.54 | 4.89 |
| Ex. 5 | 1.57 | 3.71 | 5.20 |
| Ex. 6 | 1.57 | 3.59 | 5.02 |
| Ex. 7 | 1.63 | 3.92 | 5.39 |
| Ex. 8 | 1.44 | 3.16 | 4.46 |
| Ref. Ex. 9 | 1.55 | 3.46 | 4.82 |
| Ref. Ex. 10 | 1.30 | - | 3.01 |

Each formulation was also subjected to an additional initial drying test, as well as determining film formation and a drying test following accelerated aging for four weeks at 50°C. In addition, film formation was determined following two months at room temperature. The results are provided in Table 4

**Table 4: Additional results for Example 1**

| **Formulation** | **Initial Stage IV Dry Time (hr)** | **Post-aging Stage IV Dry Time (hr)** | **Skin Formation - 2 mo @ RT** | **Skin Formation - 4 wk @ 50°C** | **Dry skin thickness after aging 4wk @ 50°C (mm)** |
|---|---|---|---|---|---|
| Ex. 1 | 5.46 | 11.74 | None | Yes | 0.3 |
| Ex. 2 | 5.27 | 11.11 | None | Yes | 0.25 |
| Ex. 3 | 5.62 | 12.42 | None | Yes | 0.34 |
| Ex. 4 | 4.87 | 12.77 | None | Yes | 0.47 |
| Ex. 5 | 5.24 | 9.73 | None | None | Easily redispersible |
| Ex. 6 | 5.04 | 9.86 | None | None | Easily redispersible |
| Ex. 7 | 5.43 | 11.19 | None | None | Easily redispersible |
| Ex. 8 | 4.48 | 11.27 | None | None | Easily redispersible |
| Ref. Ex. 9 | 4.84 | 10.13 | None | None | Easily redispersible |
| Ref. Ex. 10 | 3.05 | N/A | Yes (solid) | Yes | N/A |

The Ref. Ex. 10 formulation, having no anti-skinning agent, was completely solid following two months at room temperature.

As shown in Tables 3 and 4, the Ex. 1-8 formulations had relatively similar initial dry times to the Ref. Ex. 9 MEKO formulation. The Ex. 5-8 formulations, having a 2-PO purity of at least 98 wt.% 2-PO had no skin formation following the accelerated aging test.

Example 1 illustrates that compositions having a purity of at least 92 wt.% 2-PO are effective anti-skinning agents and can function as a substitute anti-skinning agent for MEKO.

b) Example 2A: Glossy white one-coat finish with cobalt-based drier

Glossy white one-coat finish = EU SF 3.11 //cobalt drier formulations at 0.25 wt.% anti-skinning agent, 0.35 wt.% anti-skinning agent, and 0.5 wt.% anti-skinning agent were prepared according to the weight percentages shown in Table 5. The amount of the solvent white spirit D60 added in Part B and the anti-skinning agent added in Part C for each formulation are provided in Table 6.

**Table 5: Example 2A formulations (wt.%)**

| **Part** | **Component** | **Function** | **Wt.%** |
|---|---|---|---|
| A | SETAL AF 681 TB | Alkyd resin | 30 |
| A | White spirit D60 | Solvent | 10 |
| A | Borchi Gen 911 | Wetting and dispersing agent | 2.6 |
| A | Kronos 2310 | Titanium dioxide | 26 |
| A | Borchi Gol E, 50% in Solvesso 100 | Flow promoter and release agent | 1 |
| A | Octa-Soligen Calcium 10, basic | Calcium-containing drier | 0.5 |
| B | SETAL AF 681 TB | Alkyd resin | 20 |
| B | White spirit D60 | Solvent | See Table 6 |
| B | Borchi Gol OL 17, 10% in xylene | Flow promoter and release agent | 1 |
| B | Octa-Soligen Zirconium 12 | Zirconium-containing drier | 0.5 |
| B | Octa-Soligen 69 | Cobalt-containing drier | 0.3 |
| C | Anti-skinning agent | Anti-skinning agent | See Table 6 |

**Table 6: Anti-skinning agent composition (wt.%)**

| **Formulation** | **Part B White spirit D60** | **MEKO** | **2-PO** | **3-methyl-2-butanone oxime** |
|---|---|---|---|---|
| Ref. Ex. 11 | 7.85 | 0.25 | - | - |
| Ref. Ex. 12 | 7.75 | 0.35 | - | - |
| Ref. Ex. 13 | 7.60 | 0.50 | - | - |
| Ex. 14 | 7.85 | - | 0.25 | - |
| Ex. 15 | 7.75 | - | 0.35 | - |
| Ex. 16 | 7.60 | - | 0.50 | - |
| Ex. 17 | 7.85 | - | - | 0.25 |
| Ex. 18 | 7.75 | - | - | 0.35 |
| Ex. 19 | 7.60 | - | - | 0.50 |

The MEKO was provided as a 100% MEKO composition. The 2-PO was provided as a >99.9 wt.% 2-PO composition. The 3-methyl-2-butanone oxime was provided as a 100% 3-methyl-2-butanone oxime composition.

The Part A components were subjected to ball milling at 3500 rpm for 45 minutes prior to cooling. The Part B components were then incorporated under gentle agitation/homogenization for 5 minutes.

The Part C anti-skin additive for each formulation was varied according to the formulations provided in Table 6. The Part C anti-skin additive was incorporated into Part A and Part B seven days following preparation of Part A and Part B for Ref. Ex. 11-13 and Ex. 14-15, and eight days following preparation for Ex. 16-19.

Each formulation was subjected to an additional initial drying test, in which the times for Stage I "Set-to-Touch," Stage II "Tack-free," and Stage IV "Dry-through" were determined. The results are provided in Table 7.

**Table 7: Initial drying times for Example 2A**

| **Formulation** | **Stage I Dry Time (hr)** | **Stage II Dry Time (hr)** | **Stage IV Dry Time (hr)** |
|---|---|---|---|
| Ref. Ex. 11 | 1.52 | 2.86 | 3.47 |
| Ref. Ex. 12 | 1.43 | 2.98 | 4.13 |
| Ref. Ex. 13 | 1.49 | 3.27 | 4.42 |
| Ex. 14 | 1.49 | 3.32 | 4.28 |
| Ex. 15 | 1.52 | 3.65 | 4.89 |
| Ex. 16 | 1.37 | 3.20 | 4.57 |
| Ex. 17 | 1.45 | 2.88 | 3.89 |
| Ex. 18 | 1.51 | 2.87 | 3.78 |
| Ex. 19 | 1.53 | 3.14 | 4.46 |

Each formulation was also subjected to an additional initial drying test, as well as determining film formation and a drying test following accelerated aging for four weeks at 50°C. In addition, film formation was determined following two months at room temperature. The results are provided in Table 8

**Table 8: Additional results for Example 2A**

| **Formulation** | **Initial Stage IV Dry Time (hr)** | **Post-aging Stage IV Dry Time (hr)** | **Skin Formation-2 mo @ RT** | **Skin Formation-4 wk @ 50°C** | **Dry skin thickness after aging 4wk @ 50°C (mm)** |
|---|---|---|---|---|---|
| Ref. Ex. 11 | 3.50 | 7.58 | None | Yes | 0.25 |
| Ref. Ex. 12 | 4.15 | 10.39 | None | Yes | 0.40 |
| Ref. Ex. 13 | 4.45 | >13.7 | None | Yes | 0.52 |
| Ex. 14 | 4.31 | 7.31 | None | Yes | 0.22 |
| Ex. 15 | 4.93 | 10.00 | None | Yes | 0.25 |
| Ex. 16 | 4.60 | >13.7 | None | Yes | 0.67 |
| Ex. 17 | 3.92 | 8.32 | Yes | Yes | 0.27-0.60 |
| Ex. 18 | 3.81 | 13.25 | None | Yes | 0.35 |
| Ex. 19 | 4.48 | 13.23 | None | Yes | 0.62 |

As shown in Tables 7 and 8, the formulations containing MEKO (Ref. Ex. 11-13) had similar drying times to formulations having equivalent levels of 2-PO (Ex. 14-16) and 3-methyl-2-butanone oxime (Ex. 17-19).

Example 2A illustrates that 2-PO and 3-methyl-2-butanone oxime can function as a substitute anti-skinning agent at equivalent levels as MEKO in a glossy white one-coat finish with a cobalt-based drier.

### c) Example 2B: Glossy white one-coat finish with cobalt-free drier

Glossy white one-coat finish = EU SF 3.11 //cobalt free formulations at 0.25 wt.% anti-skinning agent, 0.35 wt.% anti-skinning agent, and 0.5 wt.% anti-skinning agent were prepared according to the weight percentages shown in Table 9. The amount of the solvent white spirit D60 added in Part B and the anti-skinning agent added in Part C for each formulation are provided in Table 10.

**Table 9: Example 2B formulations (wt.%)**

| **Part** | **Component** | **Function** | **Wt.%** |
|---|---|---|---|
| A | SETAL AF 681 TB | Alkyd resin | 30 |
| A | White spirit D60 | Solvent | 10 |
| A | Borchi Gen 911 | Wetting and dispersing agent | 2.6 |
| A | Kronos 2310 | Titanium dioxide | 26 |
| A | Borchi Gol E, 50% in Solvesso 100 | Flow promoter and release agent | 1 |
| A | Octa-Soligen Calcium 10, basic | Calcium-containing Drier | 0.5 |
| B | SETAL AF 681 TB | Alkyd resin | 20 |
| B | White spirit D60 | Solvent | See Table 6 |
| B | Borchi Gol OL 17, 10% in xylene | Flow promoter and release agent | 1 |
| B | Borchi OXY - coat | Manganese-containing drier | 1 |
| C | Anti-skinning agent | Anti-skinning agent | See Table 6 |

**Table 10: Anti-skinning agent composition (wt.%)**

| **Formulation** | **Part B White spirit D60** | **MEKO** | **2-PO** | **3-methyl-2-butanone oxime** |
|---|---|---|---|---|
| Ref. Ex. 20 | 8.37 | 0.25 | - | - |
| Ref. Ex. 21 | 8.27 | 0.35 | - | - |
| Ref. Ex. 22 | 8.12 | 0.50 | - | - |
| Ex. 23 | 8.37 | - | 0.25 | - |
| Ex. 24 | 8.27 | - | 0.35 | - |
| Ex. 25 | 8.12 | - | 0.50 | - |
| Ex. 26 | 8.37 | - | - | 0.25 |
| Ex. 27 | 8.27 | - | - | 0.35 |
| Ex. 28 | 8.12 | - | - | 0.50 |

The MEKO was provided as a 100% MEKO composition. The 2-PO was provided as a >99.9 wt.% 2-PO composition. The 3-methyl-2-butanone oxime was provided as a 100% 3-methyl-2-butanone oxime composition.

The Part A components were subjected to ball milling at 3500 rpm for 45 minutes prior to cooling. The Part B components were then incorporated under gentle agitation/homogenization for 5 minutes.

The Part C anti-skin additive for each formulation was varied according to the formulations provided in Table 6. The Part C anti-skin additive was incorporated into Part A and Part B eight days following preparation of Part A and Part B for Ref. Ex. 20-22 and Ex. 23, and nine days following preparation for Ex. 24-28.

Each formulation was subjected to an additional initial drying test, in which the times for Stage I "Set-to-Touch," Stage II "Tack-free," and Stage IV "Dry-through" were determined. The results are provided in Table 11.

**Table 11: Initial drying times for Example 2B**

| **Formulation** | **Stage I Dry Time (hr)** | **Stage II Dry Time (hr)** | **Stage IV Dry Time (hr)** |
|---|---|---|---|
| Ref. Ex. 20 | 1.43 | 2.77 | 3.37 |
| Ref. Ex. 21 | 1.25 | 2.61 | 3.27 |
| Ref. Ex. 22 | 1.48 | 2.80 | 3.66 |
| Ex. 23 | 1.38 | 2.64 | 3.86 |
| Ex. 24 | 1.41 | 2.77 | 2.54 |
| Ex. 25 | 1.46 | 2.92 | 3.69 |
| Ex. 26 | 1.42 | 2.47 | 3.49 |
| Ex. 27 | 1.52 | 2.81 | 3.92 |
| Ex. 28 | 1.32 | 2.63 | 3.54 |

Each formulation was also subjected to an additional initial drying test, as well as determining film formation and a drying test following accelerated aging for four weeks at 50°C. In addition, film formation was determined following two months at room temperature. The results are provided in Table 1

**Table 12: Additional results for Example 2B**

| **Formulation** | **Initial Stage IV Dry Time (hr)** | **Post-aging Stage IV Dry Time (hr)** | **Skin Formation - 2 mo @ RT** | **Skin Formation-4 wk @ 50°C** | **Dry skin thickness after aging 4wk @ 50°C (mm)** |
|---|---|---|---|---|---|
| Ref. Ex. 20 | 3.26 | 5.72 | None, but gel formation | Yes | 7-8 |
| Ref. Ex. 21 | 3.29 | >13.7 | None, but gel formation | None | Easily redispersible |
| Ref. Ex. 22 | 3.68 | >13.7 | None | None | Easily redispersible |
| Ex. 23 | 3.91 | 5.4 | None | Yes | 4-5 |
| Ex. 24 | 3.67 | 6.73 | None | Yes | 7 |
| Ex. 25 | 3.73 | 6.41 | None | Yes | Gelation on top part of paint |
| Ex. 26 | 3.52 | 9.20 | None | Yes | 1.3 |
| Ex. 27 | 3.96 | 6.08 | None | Yes | Gelation on top part of paint |
| Ex. 28 | 3.59 | 6.80 | None | Yes | Gelation on top part of paint |

As shown in Tables 11 and 12, the formulations containing MEKO (Ref. Ex. 20-22) had similar drying times to formulations having equivalent levels of 2-PO (Ex. 23-25) and 3-methyl-2-butanone oxime (Ex. 26-28).

Example 2B illustrates that 2-PO and 3-methyl-2-butanone oxime can function as a substitute anti-skinning agent at equivalent levels as MEKO in a glossy white one-coat finish with a cobalt-free drier.

### d) Example 3: Clear gloss base

Anti-skinning agent was added to a clear gloss base as shown in Table 13.

**Table 13: Example 3 formulations (g)**

| **Formulation** | **Clear gloss base (g)** | **MEKO (g)** | **2-PO (g)** |
|---|---|---|---|
| Ref. Ex. 29 | 36 | 0.35 | - |
| Ref. Ex. 30 | 36 | 0.5 | - |
| Ref. Ex. 31 | 36 | 0.8 | - |
| Ex. 32 | 36 | - | 0.35 |
| Ex. 33 | 36 | - | 0.5 |
| Ex. 34 | 36 | - | 0.8 |

Each formulation was subjected to an additional initial drying test, in which the times for Stage I "Set-to-Touch," Stage II "Tack-free," and Stage IV "Dry-through" were determined. The results are provided in Table 14.

**Table 14: Initial drying times for Example 3**

| **Formulation** | **Stage I Dry Time (hr)** | **Stage IV Dry Time (hr)** |
|---|---|---|
| Ref. Ex. 29 | 0.57 | 1.82 |
| Ref. Ex. 30 | 0.56 | 1.67 |
| Ref. Ex. 31 | 0.56 | 1.55 |
| Ex. 32 | 0.69 | 1.98 |
| Ex. 33 | 0.59 | 1.62 |
| Ex. 34 | 0.61 | 1.37 |

Each formulation was also subjected to an additional initial drying test, as well as determining film formation and a drying test following accelerated aging for four weeks at 50°C. In addition, film formation was determined following two months at room temperature. The results are provided in Table 12

**Table 15: Additional results for Example 3**

| **Formulation** | **Initial Stage IV Dry Time (hr)** | **Post-aging Stage IV Dry Time (hr)** | **Skin Formation-2 mo @ RT** | **Skin Formation-4 wk @ 50°C** | **Dry skin thickness after aging 4wk @ 50°C (mm)** |
|---|---|---|---|---|---|
| Ref. Ex. 29 | 1.87 | 4.39 | None | None | None, but gelation |
| Ref. Ex. 30 | 1.71 | 3.32 | None | None | None, but gelation |
| Ref. Ex. 31 | 1.59 | 3.06 | None | None | None, but gelation |
| Ex. 32 | 2.02 | - | None | None | None, but gelation |
| Ex. 33 | 1.66 | 2.37 | None | None | None, but gelation |
| Ex. 34 | 1.35 | 4.19 | None | None | None, but gelation |

As shown in Tables 14 and 15, the formulations containing MEKO (Ref. Ex. 29-31) had similar drying times to formulations having equivalent levels of 2-PO (Ex. 32-34).

Example 3 illustrates that 2-PO can function as a substitute anti-skinning agent at equivalent levels as MEKO in a clear gloss base.

### e) Example 4: Satin clear base

Anti-skinning agent was added to a satin clear base as shown in Table 16.

**Table 16: Example 4 formulations (g)**

| **Formulation** | **Satin clear base (g)** | **MEKO (g)** | **2-PO (g)** |
|---|---|---|---|
| Ref. Ex. 35 | 99.65 | 0.35 | - |
| Ref. Ex. 36 | 99.5 | 0.5 | - |
| Ref. Ex. 37 | 99.2 | 0.8 | - |
| Ex. 38 | 99.65 | - | 0.35 |
| Ex. 39 | 99.5 | - | 0.5 |
| Ex. 40 | 99.2 | - | 0.8 |

Each formulation was subjected to an additional initial drying test, in which the times for Stage I "Set-to-Touch," Stage II "Tack-free," and Stage IV "Dry-through" were determined. The results are provided in Table 17.

**Table 17: Initial drying times for Example 4**

| **Formulation** | **Stage I Dry Time (hr)** | **Stage IV Dry Time (hr)** |
|---|---|---|
| Ref. Ex. 35 | 0.60 | 2.15 |
| Ref. Ex. 36 | 0.60 | 2.52 |
| Ref. Ex. 37 | 0.58 | 2.47 |
| Ex. 38 | 0.64 | 3.01 |
| Ex. 39 | 0.62 | 2.98 |
| Ex. 40 | 0.71 | 4.06 |

Each formulation was also subjected to an additional initial drying test, as well as determining film formation and a drying test following accelerated aging for four weeks at 50°C. In addition, film formation was determined following two months at room temperature. The results are provided in Table 12

**Table 18: Additional results for Example 4**

| **Formulation** | **Initial Stage IV Dry Time (hr)** | **Post-aging Stage IV Dry Time (hr)** | **Skin Formation-2 mo @ RT** | **Skin Formation-4 wk @ 50°C** | **Dry skin thickness after aging 4wk @ 50°C (mm)** |
|---|---|---|---|---|---|
| Ref. Ex. 35 | 2.18 | 2.00 | Yes - gelation | Yes | 0.85, gelation of liquid paint |
| Ref. Ex. 36 | 2.54 | 1.84 | Yes - gelation | Yes | 1, gelation of liquid paint |
| Ref. Ex. 37 | 2.49 | 3.69 | No | Yes | N/A, gelation of liquid paint |
| Ex. 38 | 3.04 | 2.52 | Yes - gelation | Yes | 0.9, gelation of liquid paint |
| Ex. 39 | 3.01 | 3.14 | Yes - gelation | Yes | 1, gelation of liquid paint |
| Ex. 40 | 4.09 | N/A | Yes - gelation | Yes | N/A, gelation of liquid paint |

For Ex. 40, aging at 50°C induced severe modifications in the liquid paint, and the drying time determination became meaningless.

As shown in Tables 17 and 18, the formulations containing MEKO (Ref. Ex. 35-37) had similar drying times to formulations having equivalent levels of 2-PO (Ex. 38-40).

Example 4 illustrates that 2-PO can function as a substitute anti-skinning agent at equivalent levels as MEKO in a satin clear base.

## Claims

1. A coating composition comprising:
at least one solvent;
at least one alkyd resin;
at least one drier; and
an anti-skinning composition capable of preventing oxidative crosslinking of the resin to form a skin, the anti-skinning composition comprising at least 92 wt.% of an alkyl oxime based on the total weight of the anti-skinning composition, wherein the alkyl oxime is selected from 2-pentanone oxime and 3-methyl-2-butanone oxime; and
wherein the at least one drier is an oxidation catalyst selected from cobalt esters and manganese esters.

2. The coating composition of claim 1, wherein the alkyl oxime comprises 2-pentanone oxime.

3. The coating composition of claim 2, wherein the anti-skinning composition comprises at least 98 wt.% 2-pentanone oxime, based on the total weight of the anti-skinning composition.

4. The coating composition of claim 2, wherein the anti-skinning composition comprises less than 0.5 wt.% methyl isobutyl ketoxime, based on the total weight of the anti-skinning composition.

5. The coating composition of claim 2, wherein the anti-skinning composition comprises less than 0.3 wt.% methyl isobutyl ketoxime, based on the total weight of the anti-skinning composition.

6. The coating composition of claim 2, wherein the coating composition comprises less than 0.06 wt.% methyl isobutyl ketoxime, based on the total weight of the coating composition.

7. The coating composition of claim 1, wherein the solvent is present in an amount of 10 wt.% to 40 wt.% based on the total weight of the coating composition.

## Patentansprüche

1. Überzugszusammensetzung, umfassend:
mindestens ein Lösungsmittel,
mindestens ein Alkydharz,
mindestens ein Trockenmittel und
eine Anti-Hautbildung-Zusammensetzung, die in der Lage ist, die oxidative Vernetzung des Harzes unter Bildung einer Haut zu verhindern, wobei die Anti-Hautbildungszusammensetzung mindestens 92 Gew.-% eines Alkyloxims, bezogen auf das Gesamtgewicht der Anti-Hautbildungszusammensetzung, umfasst, wobei das Alkyloxim aus 2-Pentanonoxim und 3-Methyl-2-butanonoxim ausgewählt ist, und
wobei es sich bei dem mindestens einen Trockenmittel um einen aus Kobaltestern und Manganestern ausgewählten Oxidationskatalysator handelt.

2. Überzugszusammensetzung nach Anspruch 1, wobei das Alkyloxim 2-Pentanonoxim umfasst.

3. Überzugszusammensetzung nach Anspruch 2, wobei die Anti-Hautbildungszusammensetzung mindestens 98 Gew.-% 2-Pentanonoxim, bezogen auf das Gesamtgewicht der Anti-Hautbildungszusammensetzung, umfasst.

4. Überzugszusammensetzung nach Anspruch 2, wobei die Anti-Hautbildungszusammensetzung weniger als 0,5 Gew.-% Methylisobutylketoxim, bezogen auf das Gesamtgewicht der Anti-Hautbildungszusammensetzung, umfasst.

5. Überzugszusammensetzung nach Anspruch 2, wobei die Anti-Hautbildungszusammensetzung weniger als 0,3 Gew.-% Methylisobutylketoxim, bezogen auf das Gesamtgewicht der Anti-Hautbildungszusammensetzung, umfasst.

6. Überzugszusammensetzung nach Anspruch 2, wobei die Überzugszusammensetzung weniger als 0,06 Gew.-% Methylisobutylketoxim, bezogen auf das Gesamtgewicht der Überzugszusammensetzung, umfasst.

7. Überzugszusammensetzung nach Anspruch 1, wobei das Lösungsmittel in einer Menge von 10 Gew.-% bis 40 Gew.-%, bezogen auf das Gesamtgewicht der Zusammensetzung, vorhanden ist.

## Revendications

1. Composition de revêtement comprenant :
au moins un solvant ;
au moins une résine alkyde ;
au moins un agent desséchant ; et
une composition anti-peau capable de prévenir la réticulation oxydative de la résine pour former une peau, la composition anti-peau comprenant au moins 92 % en poids d'une alkyloxime sur la base du poids total de la composition anti-peau, dans laquelle l'alkyloxime est choisie parmi la 2-pentanone-oxime et la 3-méthyl-2-butanone-oxime ; et
dans laquelle l'au moins un agent desséchant est un catalyseur d'oxydation choisi parmi des esters de cobalt et des esters de manganèse.

2. Composition de revêtement selon la revendication 1, dans laquelle l'alkyloxime comprend la 2-pentanone-oxime.

3. Composition de revêtement selon la revendication 2, la composition anti-peau comprenant au moins 98 % en poids de 2-pentanone-oxime, sur la base du poids total de la composition anti-peau.

4. Composition de revêtement selon la revendication 2, la composition anti-peau comprenant moins de 0,5 % en poids de méthylisobutylcétoxime, sur la base du poids total de la composition anti-peau.

5. Composition de revêtement selon la revendication 2, la composition anti-peau comprenant moins de 0,3 % en poids de méthylisobutylcétoxime, sur la base du poids total de la composition anti-peau.

6. Composition de revêtement selon la revendication 2, la composition de revêtement comprenant moins de 0,06 % en poids de méthylisobutylcétoxime, sur la base du poids total de la composition de revêtement.

7. Composition de revêtement selon la revendication 1, dans laquelle le solvant est présent en une quantité de 10 % en poids à 40 % en poids sur la base du poids total de la composition.
